# FASCICULE DE BREVET EUROPEEN

(11) **EP 0 876 808 B1**
(45) Date de publication et mention de la délivrance du brevet: **01.10.2003**
(21) Numéro de dépôt: 98108134.2
(22) Date de dépôt: 05.05.1998
(51) Int. Cl.: A61F 5/00

(54) **Dispositif à bande gastrique réglable pour resserrer l'estomac d'un patient**
Einstellbares Magenband zum Festspannen des Magens eines Patienten
Adjustable gastric band device for tightening the stomach of a patient

(30) Priorité: 07.05.1997 EP 97810287
(43) Date de publication de la demande: 11.11.1998
(73) Titulaire: Klasamed S.A., 2006 Neuchâtel (CH)
(72) Inventeur: Klaiber, Christian, 3270 Aarberg (CH); de Salis, Sker, 2006 Neuchâtel (CH); Siegenthaler, Stephan, 3427 Utzenstorf (CH)
(74) Mandataire: Mercer, Christopher Paul

(56) Documents cités:
- EP-A- 0 202 815
- GB-A- 1 174 814
- US-A- 3 750 194
- US-A- 3 863 622
- US-A- 5 449 368

## Description

La présente invention concerne un dispositif à bande gastrique réglable pour resserrer l'estomac d'un patient, comportant une bande gastrique destinée à être implantée autour de l'estomac du patient et ayant une cavité à volume variable remplie d'un liquide, et des moyens de réglage du volume de liquide dans ladite cavité, les moyens de réglage comprenant une partie implantable dans le corps du patient et raccordée à la cavité de la bande gastrique par un tuyau.

Un tel dispositif est utilisable dans une méthode connue de lutte contre l'obésité, appelé "Adjustable Gastric Banding", consistant à étrangler pendant plusieurs mois ou plusieurs années une partie supérieure de l'estomac du patient au moyen d'une bande gastrique implantée pour former dans l'estomac un passage (stoma) de diamètre réduit qui limite la vitesse d'ingestion des aliments. On peut ajuster ce diamètre en fonction de l'évolution du patient, en enlevant ou ajoutant du liquide dans la cavité à volume variable de la bande gastrique.

Des bandes gastriques de ce genre sont décrites dans les brevets US 4 592 339, 4 696 288, 5 074 868 et 5 449 368, et dans les demandes internationales WO 86/04498 et WO 94/27504. Elles sont utilisables dans le cadre de la présente invention.

Le dispositif décrit dans le brevet US 4 592 339 comporte habituellement, en guise de boîte de commande pour le réglage du volume de liquide, une unité d'injection implantée directement sous la peau du patient et pourvue d'une membrane auto-obturante. L'intérieur de cette unité est raccordé à la bande gastrique par un tuyau souple. En piquant l'aiguille d'une seringue à travers la peau et la membrane, le médecin peut injecter un supplément de liquide ou au contraire en retirer, pour modifier l'ouverture du passage dans l'estomac selon les besoins.

Le principal inconvénient de ces dispositifs de l'art antérieur est que, pour ajuster le volume de liquide dans la bande gastrique, on doit procéder depuis l'extérieur à des prélèvements ou des adjonctions de liquide au moyen d'une seringue, ce qui occasionne à chaque fois une piqûre, de l'inconfort et un risque d'infection pour le patient. D'autre part, le risque de fuite du liquide à travers la membrane auto-obturante n'est pas nul. Un autre risque est qu'une personne non autorisée pourrait, à l'aide d'une simple seringue, modifier le volume de liquide à l'insu du médecin traitant et donc perturber le traitement.

La présente invention a pour but de remédier à cet inconvénient, en créant un dispositif à bande gastrique qui est réglable d'une manière non invasive et sans inconfort pour le patient. Un but particulier consiste à créer un dispositif qui présente à l'usage une sécurité accrue, en permettant notamment de surveiller son fonctionnement d'une manière non invasive, de donner des signaux d'alarme et d'empêcher des interventions non autorisées.

Dans sa forme générale, l'invention concerne un dispositif à bande gastrique réglable du genre indiqué en préambule, caractérisé en ce que la partie implantable des moyens de réglage comporte un réservoir, raccordé à ladite cavité via le tuyau, et un ensemble de commande comportant une batterie électrique, une unité électronique de commando et une pompe électrique raccordée au réservoir et à la batterie et commandée par l'unité de commande, pour transférer du liquide en circuit fermé entre le réservoir et la cavité de la bande gastrique via le tuyau, et en ce que les moyens de réglage comprennent en outre un appareil de contrôle, situe à l'extérieur du corps du patient et ayant des moyens de communication sans fil pour transmettre des signaux à l'unité de commande.

Ainsi, le dispositif selon l'invention peut fonctionner durant toute sa durée de service avec un volume de liquide constant, les ajustements étant effectués par des transferts de volumes prédéterminés de liquide en circuit fermé entre la bande gastrique et le réservoir au moyen d'une boîte de commande qui est implantée dans le corps comme un stimulateur cardiaque et qui, le cas échéant, peut être télécommandée d'une manière non invasive à travers la peau du patient. L'expression "circuit fermé" met en évidence le fait qu'un volume invariable de liquide est contenu dans un ensemble clos de chambres et de conduits formant un circuit hydrostatique sans communication de fluide avec l'extérieur de cet ensemble, lequel est implanté entièrement dans le corps du patient.

L'utilisation d'une pompe raccordée à un réservoir de compensation et implantée dans le corps humain a déjà été proposée pour ouvrir et fermer un sphincter artificiel. Par exemple, dans le brevet US 3 750 194, il est décrit une pompe non motorisée ayant un rotor aimanté qu'on fait tourner par entraînement magnétique à partir de l'extérieur du corps pour fermer de manière réversible un passage naturel ou implanté dans le corps, notamment un passage urinaire ou un canal alimentaire. Toutefois un tel dispositif n'est pas transposable au bandage gastrique, parce qu'il fonctionne en "tout ou rien", c'est-à-dire qu'il ferme ou ouvre le passage corporel, mais ne peut pas ajuster un degré d'ouverture de ce passage. Au contraire, la méthode de bandage gastrique exige que le passage dans l'estomac soit toujours ouvert, dans une mesure précise et constante, et que le réglage de cette ouverture soit fait par le transfert d'une quantité connue de liquide.

L'utilisation d'une pompe motorisée, de préférence à déplacement positif, associée à une source d'énergie électrique et à une unité électronique de commande dans un ensemble implanté dans le corps constitue un moyen sûr pour maîtriser les volumes transférés par la pompe, donc aussi la taille du passage dans l'estomac. Or cette taille et les variations que le médecin lui impose sont les paramètres essentiels du traitement par bandage gastrique. De plus, il faut être sûr que le passage gastrique ne soit jamais fermé.

De préférence, les moyens de réglage comprennent un appareil de contrôle, dit aussi contrôleur, situé à l'extérieur du corps du patient, et l'unité de commande et l'appareil de contrôle comportent des moyens de communication sans fil, notamment en radiofréquences, pour transmettre des informations entre eux. Un médecin peut ainsi télécommander la pompe par le contrôleur pour transférer du liquide entre la bande gastrique et le réservoir. En outre il peut transmettre des informations et des ordres à l'unité de commande et en recevoir des informations pour vérifier son bon fonctionnement.

En particulier, l'unité de commande est agencée de préférence de façon à transmettre par lesdits moyens de communication sans fil une indication du volume de liquide transféré. L'appareil de contrôle reçoit ainsi un "feedback" du système implanté dans le corps, permettant de surveiller son fonctionnement, d'enregistrer avec précision les volumes transférés et de connaître ainsi l'état du système en tout temps, en particulier le volume du liquide présent dans la bande gastrique.

Dans une forme de réalisation préférée, la boîte de commande comporte un capteur de pression indiquant à l'unité de commande la pression du liquide dans le tuyau, et l'unité de commande est agencée pour produire un signal d'alarme lorsque ladite pression est sortie d'une gamme prédéterminée. Les valeurs limites de cette gamme sont enregistrées dans une mémoire de l'unité de commande et peuvent être modifiées par radio au moyen du contrôleur.

Le dispositif selon l'invention peut comprendre en outre un moniteur situé à l'extérieur du corps du patient et pourvu de moyens de communication sans fil avec l'unité de commande, le moniteur ayant des moyens d'affichage qui sont activés en réponse à la réception dudit signal d'alarme.

Dans une forme d'exécution particulière, la pompe est susceptible de fonctionner sélectivement dans des sens opposés, pour pomper du liquide soit en direction de la bande gastrique, soit en direction du réservoir.

D'autres caractéristiques et avantages de la présente invention apparaîtront dans la description suivante d'un mode de réalisation préféré, présenté à titre d'exemple non limitatif en référence aux dessins annexés, dans lesquels :
- la figure 1 est une vue schématique d'ensemble du dispositif selon l'invention;
- la figure 2 est une vue en perspective de la bande gastrique fixée sur l'estomac;
- la figure 3 est un schéma-bloc de la boîte de commande, et
- la figure 4 est un schéma représentant une variante de la réalisation représentée à la figure 3.

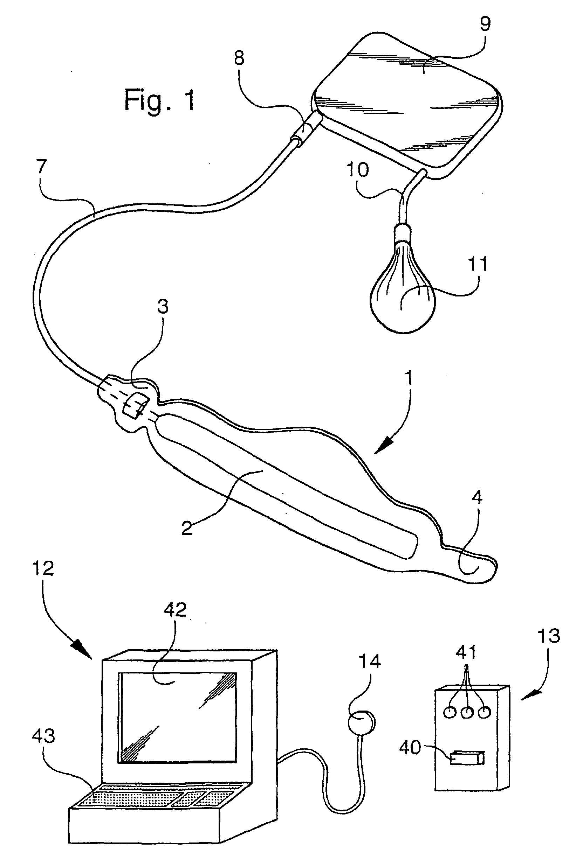

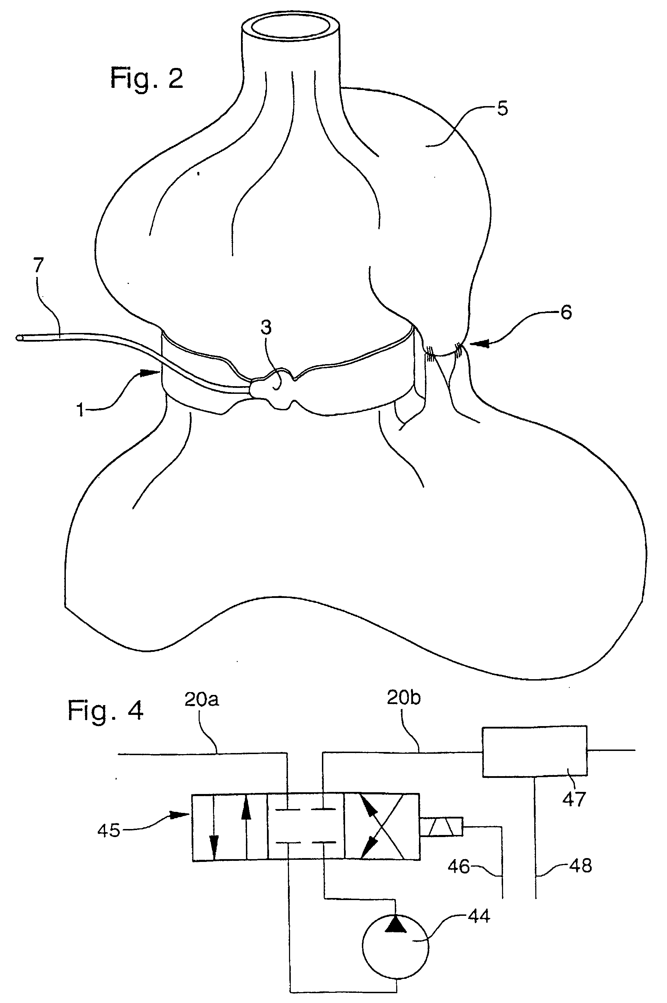

La figure 1 représente l'ensemble des constituants principaux du dispositif selon l'invention. Une bande gastrique 1, qui peut être par exemple de l'un des types décrits dans les brevets antérieurs mentionnés plus haut, comporte sur sa face intérieure une paroi souple qui délimite une cavité 2 remplie d'un volume variable de liquide. Les extrémités 3 et 4 de la bande 1 sont pourvues d'organes d'attache de type connu, permettant de fixer ces extrémités l'une à l'autre une fois que la bande 1 a été placée autour d'une partie supérieure 5 de l'estomac du patient, comme le montre la figure 2. De préférence, une suture 6 est pratiquée sur la paroi extérieure de l'estomac pour maintenir la bande en place.

On voit dans la figure 1 que l'extrémité 3 de la bande 1 est raccordée à un tuyau souple 7 dont l'autre extrémité est connectée par un raccord auto-obturant 8 à une boîte de commande 9. Cette boîte est aussi raccordée par un tuyau 10 à un réservoir de compensation 11 qui contient aussi un volume variable de liquide et qui communique avec la cavité 2 de la bande 1 à travers le tuyau 10, la boîte 9 et le tuyau 7. La boîte 9 contient des moyens permettant de transférer du liquide du réservoir 11 à la cavité 2 et vice versa.

Le réservoir 11 est réalisé de préférence sous la forme d'une poche souple placée à proximité de la boîte de commande 9. Il pourrait aussi être placé directement sur une face de cette boîte ou à l'intérieur de la boîte.

La boîte de commande 9 et le réservoir 11 sont implantés dans le corps du patient dans un endroit approprié, qui peut être éloigné de l'estomac, par exemple directement sous la peau du patient afin que la boîte 9 soit facilement accessible au besoin. Etant donné que cette boîte contient des moyens électromagnétiques de communication avec l'extérieur, comme on le décrira plus loin, une implantation sous-cutanée est préférable et permet aussi de remplacer facilement la boîte et le réservoir s'il le faut.

Les autres composants du dispositif représenté à la figure 1 sont un contrôleur 12 et un moniteur 13 qui se trouvent à l'extérieur du corps du patient. Chacun de ces deux appareils est agencé pour communiquer avec la boîte de commande 9 par voie électromagnétique, par exemple par radio. Le contrôleur 12 est destiné au médecin traitant. Il peut présenter l'aspect d'un micro-ordinateur équipé d'un émetteur-récepteur radio ayant une antenne 14 qu'on peut fixer sur le corps du patient en regard de la boîte 9. Le moniteur 13, ayant approximativement la taille d'une boîte d'allumettes, est destiné au patient, qui le portera sur lui ou le conservera à proximité. Il peut communiquer avec la boîte 9 par un système de transmission analogue à celui du contrôleur 12. On notera toutefois que le moniteur 13 n'est pas un élément essentiel dans le mode de réalisation décrit ici.

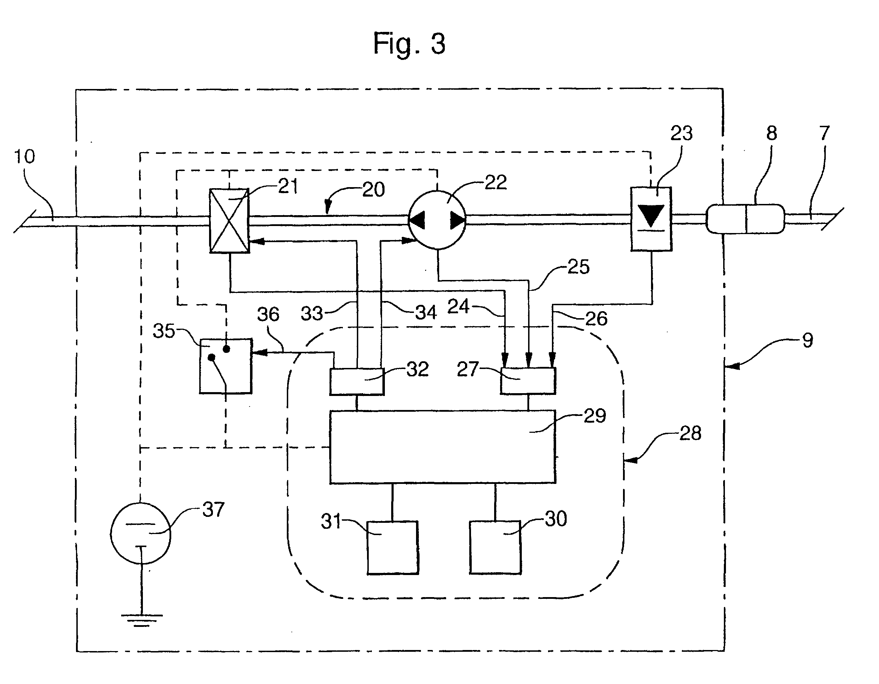

La figure 3 représente schématiquement les principaux éléments fonctionnels contenus dans la boîte de commande 9. Le tuyau 10 provenant du réservoir 11 est relié au raccord 8 du tuyau 7 par un circuit de liquide 20 sur lequel sont montés en série une électrovanne 21, une pompe à moteur électrique 22 et un capteur de pression 23. Ces trois éléments sont reliés par des lignes électriques 24, 25 et 26 à des entrées 27 d'une unité électronique de commande 28 comportant un circuit logique 29, constitué par exemple par un circuit intégré tel qu'un microprocesseur et couplé à une mémoire externe 30 et à un émetteur-récepteur radio 31 pourvu d'une antenne non représentée. L'unité de commande 28 comporte en outre des sorties 32 reliées à l'électrovanne 21 et à la pompe 22 par des lignes électriques de commande 33 et 34, et à un relais 35 par une ligne électrique de commande 36.

La pompe 22 est de préférence une pompe volumétrique capable de pomper dans les deux sens, par exemple une pompe péristaltique. Le sens de fonctionnement peut changer simplement par inversion de la polarité de l'alimentation du moteur.

La boîte de commande 9 contient en outre une pile électrique 37 qui alimente, par des lignes représentées en trait interrompu, l'unité de commande 28 et le capteur de pression 23, ainsi que l'électrovanne 21 et la pompe 22 à travers le relais 35 commandé par l'unité 28. Ce relais est habituellement ouvert afin d'économiser de l'énergie; il n'est fermé que lorsque l'électrovanne 21 et/ou la pompe 22 doivent être actionnés.

L'unité de commande 28 reçoit par la ligne 24 un signal indiquant si l'électrovanne 21 est ouverte ou fermée, par la ligne 25 un signal indiquant si la pompe 22 est arrêtée ou en marche, et dans quel sens, et par la ligne 26 un signal indiquant la pression du liquide dans le tuyau 7 et donc dans la cavité 2 du bande gastrique 1. L'unité 28 commande par la ligne 33 l'ouverture et la fermeture de l'électrovanne 21, par la ligne 34 la mise en marche, le sens de pompage et l'arrêt de la pompe 22, et par la ligne 36 la fermeture et l'ouverture du relais 35.

L'état normal de la boîte de commande 9 est un état de veille. L'électrovanne 21 est fermée et la pompe 22 est arrêtée, de sorte que les volumes de liquide respectifs dans la cavité 2 de la bande gastrique et dans le réservoir 11 sont invariables. Le relais 35 étant ouvert, seuls l'unité 28 et le capteur de pression 23 sont alimentés par la pile 37. L'émetteur-récepteur 31 est prêt à la réception. L'unité 28 surveille en permanence les paramètres essentiels qui sont :
- la pression dans le circuit du liquide, cette pression devant rester entre une valeur minimale et une valeur maximale qui sont prédéterminées et enregistrées dans une partie programmable de la mémoire 30;
- la tension de la pile 37;
- la présence d'un éventuel court-circuit;
- le résultat d'un programme d'auto-diagnostic.

En outre, l'unité 28 émet à des intervalles périodiques, par exemple deux fois par jour, par l'émetteur-récepteur 31, un signal d'interrogation destiné à vérifier si le moniteur 13 est à portée de transmission radio et en état de fonctionner, afin d'inciter le patient à garder le moniteur sur lui. Si par exemple deux interrogations sont infructueuses, le moniteur 13 est programmé pour émettre une alarme acoustique qui pourra être répétée jusqu'à ce que le patient ou son entourage réagisse : en ramenant le moniteur à proximité du patient, on pourra envoyer un signal du moniteur vers la boîte de commande 9 pour rétablir la liaison, ce qui fera cesser l'alarme.

Dans la figure 1, on notera que le moniteur est équipé d'un bouton de commande 40, pour produire l'émission d'un signal radio à destination de la boîte 9, et de trois diodes luminescentes 41 servant de témoins de fonctionnement et d'alarme.

Si la surveillance exercée par l'unité de commande 28 détecte une anomalie dans les paramètres essentiels mentionnés plus haut, l'unité 28 va produire les actions suivantes :
1. Activation du mode d'émission de l'émetteur-récepteur 31.
2. Emission d'un bref signal pour activer dans le moniteur 13 un code d'alarme correspondant au paramètre défectueux.
3. Les différentes alarmes possibles sont affichées sur le moniteur 13 au moyen de combinaisons des diodes 41.
4. Le moniteur 13 donne quittance de la réception de l'alarme à l'unité de commande 28. La combinaison de diodes restera allumée jusqu'à la remise à l'état initial du moniteur 13 par le contrôleur 12.
5. Remise en mode de réception de l'émetteur-récepteur 31.

Cette séquence est agencée pour réduire au maximum les durées d'émission de l'émetteur-récepteur 31, afin d'économiser l'énergie de la pile 37, tout en assurant une sécurité optimale du fonctionnement du dispositif.

Le moniteur 13 contient aussi de préférence un microprocesseur et dispose d'un système d'auto-diagnostic, indépendant de la boîte de commande 9, notamment pour surveiller l'état de sa pile, que le patient peut facilement changer lui-même.

Lorsque le patient visite son médecin, soit parce que son moniteur 13 a signalé une alarme, soit pour un contrôle de routine, le médecin utilise le contrôleur 12 pour intervenir sur la partie du dispositif implantée dans le corps du patient. Plusieurs types d'intervention sont possibles.

Tout d'abord, le contrôleur 12 va identifier le patient. A cet effet, chaque unité de commande 28 contient, dans la partie non programmable et donc non modifiable de la mémoire 30, un code d'identification personnel (PIN) connu du médecin et du patient. Ce code peut en outre être gravé sur le boîtier du moniteur 13. La séquence d'identification est la suivante :
1. Le médecin introduit le PIN dans le contrôleur 12 et lance le processus d'identification.
2. Le contrôleur 12 communique le PIN à l'unité 28.
3. L'unité 28 compare le PIN reçu à celui qu'elle contient en mémoire.
4. L'unité 28 communique au contrôleur 12 le résultat de la comparaison.

Si le résultat est positif, le médecin peut commencer l'intervention. Les opérations suivantes A à E peuvent être réalisées :

### A. Confirmation d'alarme

Le contrôleur 12 demande à l'unité 28 de répéter la dernière alarme qu'elle a donnée. Sur cette base, le médecin décide de la suite de l'intervention. Par exemple si la pile 37 est usée, il changera la boîte de commande.

### B. Modification des valeurs limites de pression

Le contrôleur 12 communique à l'unité 28 les nouvelles valeurs limites, et l'unité 28 les enregistre dans la mémoire 30 et émet une quittance.

### C. Transfert de liquide

Le contrôleur 12 communique à l'unité 28 la quantité et le sens du transfert. L'unité 28 déclenche alors la séquence suivante :
1. Mise sous tension de l'électrovanne 21 et de la pompe 22 par le relais 35.
2. Ouverture de l'électrovanne 21.
3. Mise en marche de la pompe 22 dans le sens voulu et pour la durée nécessaire, correspondant au volume à transférer. En même temps, le médecin peut vérifier la taille du passage dans l'estomac au moyen d'un gastrosténomètre introduit via l'oesophage.
4. Arrêt de la pompe 22.
5. Fermeture de l'électrovanne 21.
6. Mise hors tension de l'électrovanne et de la pompe.
7. Communication d'une quittance de transfert terminé au contrôleur. En plus, cette communication peut englober une indication du volume de liquide transféré.

### D. Mise en veille

Le contrôleur 12 transmet un signal de fin d'intervention. L'unité 28 effectue alors la séquence de diagnostic suivante vérifiant les points 2 à 6 ci-dessous.
1. Mise de l'émetteur-récepteur en mode d'émission.
2. Relais 37 ouvert.
3. Electrovanne 31 fermée.
4. Pompe 22 à l'arrêt.
5. Pression mesurée par le capteur 23 dans les limites enregistrées.
6. Etat de la pile 37.
7. Transmission d'un signal de diagnostic réussi ou raté.
8. Mise de l'émetteur-récepteur 31 en mode de réception.

Le médecin peut ainsi prendre une décision en fonction du résultat du diagnostic.

### E. Préparation pour changer la boîte de commande

Le changement de la boîte de commande 9 nécessite une intervention chirurgicale locale avec incision de la peau du patient. Pour éviter tout problème au moment du débranchement du raccord 8 et de la manipulation de la boîte 9 et du réservoir 11, le contrôleur 12 bloque toutes les fonctions de la boîte de commande 9, sauf la fonction de réception de l'émetteur-récepteur 31 (pour permettre un déblocage si le blocage est dû à une erreur).

La structure du moniteur 13 représenté à la figure 1 n'est pas décrite ici en détails, car un homme du métier peut le réaliser sans difficulté sur la base des fonctions décrites dans la présente demande. Bien entendu, il contient un émetteur-récepteur radio pour communiquer avec la boîte de commande 9. Son fonctionnement s'effectue sous le contrôle du même code d'identification personnel (PIN) que la boîte de commande 9, pour éviter tout mélange entre patients.

Le contrôleur 12 comporte de préférence un écran 42 pour afficher des informations, un clavier 43 pour l'entrée de données et la commande des fonctions, et des mémoires suffisantes pour enregistrer pour chaque patient ses données personnelles, son code d'identification personnel, les valeurs limites de pression enregistrées dans la boîte de commande 9, les transferts de liquide effectués, les dates et la nature des interventions. Il peut aussi communiquer par radio avec le moniteur 13 pour le remettre à l'état initial après réception d'une ou plusieurs alarmes. Le contrôleur 12 peut aussi comporter une interface pour être couplé à un ordinateur, par exemple afin d'y mémoriser des données, ou être directement incorporé à un ordinateur.

La présente invention n'est pas limitée au mode de réalisation décrit ci-dessus, mais elle peut faire l'objet de multiples modifications ou variantes. Par exemple, la pompe bidirectionnelle 22 représentée à la figure 3 peut être remplacée par l'agencement représenté à la figure 4, comportant une pompe électrique unidirectionnelle 44. Le conduit 20a relié au réservoir 11 et le conduit 20b relié à la bande gastrique 1 sont raccordés sélectivement à la pompe 44 par l'intermédiaire d'un distributeur hydraulique 45 à trois positions ou par un agencement de soupapes équivalent, commandé par l'unité 28 via une ligne électrique 46. Dans sa position neutre représentée à la figure 4, le distributeur 45 ferme les conduits 20a et 20b, de sorte qu'il remplace l'électrovanne 21. Ses deux autres positions permettent le transfert de liquide par la pompe 44, respectivement dans un sens et dans l'autre.

Dans la figure 4, on a représenté en outre un débitmètre 47 associé au conduit 20b et relié par une ligne électrique 48 à l'unité 28 pour indiquer à celle-ci le débit du liquide transféré par la pompe 44. Un tel débitmètre peut aussi être installé sur le circuit 20 dans le cas de la figure 3. Il est nécessaire lorsque la pompe 22 ou 44 n'est pas volumétrique, mais il peut aussi être prévu en combinaison avec une pompe volumétrique.

## Revendications

1. Dispositif à bande gastrique réglable pour resserrer l'estomac d'un patient, comportant une bande gastrique (1) destinée à être implantée autour de l'estomac du patient et ayant une cavité à volume variable (2) remplie d'un liquide, et des moyens de réglage du volume de liquide dans ladite cavité, les moyens de réglage comprenant une partie implantable dans le corps du patient et raccordée à la cavité (2) de la bande gastrique par un tuyau (7),
**caractérisé en ce que** la partie implantable des moyens de réglage comporte un réservoir (11), raccordé à ladite cavité via le tuyau (7), et un ensemble de commande comportant une batterie électrique (37), une unité électronique de commande (28) et une pompe électrique (22, 44) raccordée au réservoir (11) et à la batterie (37) et commandée par l'unité de commande (28) pour transférer du liquide en circuit fermé entre le réservoir (11) et la cavité (2) de la bande gastrique (1) via le tuyau (7), et **en ce que** les moyens de réglage comprennent en outre un appareil de contrôle (12), situé à l'extérieur du corps du patient et ayant des moyens de communication sans fil (14) pour transmettre des signaux à l'unité de commande (28).

2. Dispositif selon la revendication 1, **caractérisé en ce que** la pompe (22, 44) est une pompe volumétrique.

3. Dispositif selon la revendication 1 ou 2, **caractérisé par** une boîte de commande (9) implantable contenant l'unité électronique de commande (28) et la pompe (22, 44).

4. Dispositif selon la revendication 3, **caractérisé en ce que** la boîte de commande contient la batterie (37).

5. Dispositif selon l'une des revendications précédentes, **caractérisé par** des moyens (47) pour mesurer le volume du liquide transféré par la pompe.

6. Dispositif selon la revendication 1, **caractérisé en ce que** l'unité de commande (28) comporte des moyens de communication sans fil (31), l'unité de commande (28) et l'appareil de contrôle (12) étant agencés pour transmettre des informations entre eux par leurs moyens de communication sans fil (31, 14).

7. Dispositif selon la revendication 6, **caractérisé en ce que** lesdites informations comprennent un code d'identification personnel (PIN) enregistré dans l'unité de commande.

8. Dispositif selon les revendications 5 et 6, **caractérisé en ce que** l'unité de commande (28) est agencée pour transmettre par lesdits moyens de communication sans fil (31, 14) une indication du volume de liquide transféré.

9. Dispositif selon l'une des revendications précédentes, **caractérisé par** un capteur de pression (23) implantable, indiquant à l'unité de commande (28) la pression du liquide dans le tuyau (7), l'unité de commande (28) étant agencée pour produire un signal d'alarme via des moyens de communication sans fil (31) lorsque ladite pression est sortie d'une gamme prédéterminée.

10. Dispositif selon la revendication 9, **caractérisé en ce qu'**il comprend en outre un moniteur (13) situé à l'extérieur du corps du patient et pourvu de moyens de communication sans fil avec l'unité de commande (28), le moniteur ayant des moyens d'affichage (41) qui sont activés en réponse à la réception dudit signal d'alarme.

11. Dispositif selon l'une des revendications précédentes, **caractérisé en ce que** la pompe (22) est susceptible de fonctionner sélectivement dans des sens opposés, pour pomper du liquide soit en direction de la bande gastrique (1), soit en direction du réservoir (11).

12. Dispositif selon l'une des revendications précédentes, **caractérisé en ce qu'**une électrovanne (21, 45) commandée par l'unité de commande est branchée en série entre le réservoir (11) et la pompe (22, 44).

## Patentansprüche

1. Magen-Band-Vorrichtung, die zum Einschnüren des Magens eines Patienten steuerbar ist, mit einem Magen-Band (1), das um den Magen des Patienten herum implantierbar ist und einen Hohlraum mit einem variablen Volumen (2) aufweist, das mit einer Flüssigkeit gefüllt ist, und einer Einrichtung zum Regeln des Flüssigkeitsvolumens in dem Hohlraum, wobei die Regelungseinrichtung ein Teil umfasst, das in den Körper des Patienten implantierbar ist und mit dem Hohlraum (2) des Magen-Bands über eine Leitung (7) verbunden ist;
**dadurch gekennzeichnet, dass** das implantierbare Teil der Regelungseinrichtung einen Behälter (11), der mit dem Hohlraum über die Leitung (7) verbunden ist, und eine Steuerungsanordnung mit einer elektrischen Batterie (37), einer elektronischen Steuereinheit (28) und einer elektrischen Pumpe (22, 44) umfasst, die mit dem Behälter (11) und mit der Batterie (37) verbunden und von der Steuereinheit (28) gesteuert ist, um die Flüssigkeit in einem geschlossenen Kreislauf zwischen dem Behälter (11) und dem Hohlraum (2) des Magen-Bandes (1) über die Leitung (7) zu fördern, und dass die Regelungseinrichtung außerdem ein Steuergerät (12) umfasst, das außerhalb des Körpers des Patienten liegt und eine drahtlose Kommunikationseinrichtung (14) zum Übertragen von Signalen an die Steuereinheit (28) aufweist.

2. Vorrichtung nach Anspruch 1, **dadurch gekennzeichnet, dass** die Pumpe (22, 44) eine Volumenpumpe ist.

3. Vorrichtung nach Anspruch 1 oder 2, **gekennzeichnet durch** ein implantierbares Steuerungsgehäuse (9), das die elektronische Steuereinheit (28) und die Pumpe (22, 44) enthält.

4. Vorrichtung nach Anspruch 3, **dadurch gekennzeichnet, dass** die Batterie (37) in dem Steuergehäuse untergebracht ist.

5. Vorrichtung nach einem der vorangegangenen Ansprüche, **gekennzeichnet durch** eine Einrichtung (47) zum Messen des Flüssigkeitsvolumens, das von der Pumpe gefördert ist.

6. Vorrichtung nach Anspruch 1, **dadurch gekennzeichnet, dass** die Steuereinheit (28) eine drahtlose Kommunikationseinrichtung (31) umfasst, wobei die Steuereinheit (28) und das Steuergerät (12) zum Übertragen von Informationen über ihre drahtlose Kommunikationseinrichtung (31, 14) ausgeführt sind.

7. Vorrichtung nach Anspruch 6, **dadurch gekennzeichnet, dass** die Informationen einen persönlichen Identifikationscode (PIN) umfassen, der in der Steuereinheit gespeichert ist.

8. Vorrichtung nach Anspruch 5 und 6, **dadurch gekennzeichnet, dass** die Steuereinheit (28) eine Information über das geförderte Flüssigkeitsvolumen über die kabellosen Kommunikationseinrichtungen (31, 14) übertragen kann.

9. Vorrichtung nach einem der vorangegangenen Ansprüche, **gekennzeichnet durch** einen implantierbaren Drucksensor, welche der Steuereinheit (28) den Druck der Flüssigkeit in der Leitung (7) anzeigt, wobei die Steuereinheit (28) zum Erzeugen eines Alarmsignals über die drahtlose Kommunikationseinrichtung (31) ausgelegt ist, wenn der Druck einen vorbestimmten Bereich überschreitet.

10. Vorrichtung nach Anspruch 9, **dadurch gekennzeichnet, dass** sie außerdem einen Monitor (13) umfasst, der außerhalb des Körpers des Patienten liegt und mit der drahtlosen Kommunikationseinrichtung mit der Steuereinheit (28) versehen ist, wobei der Monitor eine Anzeigeeinrichtung (41) aufweist, die auf den Empfang des Alarmsignals hin aktiviert wird.

11. Vorrichtung nach einem der vorangegangenen Ansprüche, **dadurch gekennzeichnet, dass** die Pumpe in umgekehrter Richtung funktionieren kann, um Flüssigkeit entweder in Richtung des Magen-Bands (1) oder in Richtung des Behälters (11) zu pumpen.

12. Vorrichtung nach einem der vorangegangenen Ansprüche, **dadurch gekennzeichnet, dass** ein Elektroventil (21, 45), das von der Steuereinheit gesteuert ist, in Reihe zwischen dem Behälter (11) und der Pumpe (22, 44) geschaltet ist.

## Claims

1. Device with adjustable gastric band for tightening the stomach of a patient, comprising a gastric band (1) intended to be implanted around the stomach of the patient and having a cavity with variable volume (2) filled with a liquid, and means for adjusting the volume of liquid in the said cavity, the adjusting means comprising a part implantable in the body of the patient and connected to the cavity (2) of the gastric band by a tube (7),
**characterized in that** the implantable part of the adjusting means comprises a reservoir (11), connected to the said cavity via the tube (7), and a control assembly comprising an electric battery (37), an electronic control unit (28) and an electric pump (22, 44) connected to the reservoir (11) and to the battery (37) and controlled by the control unit (28) so as to transfer liquid in closed circuit between the reservoir (11) and the cavity (2) of the gastric band (1) via the tube (7), and **in that** the adjusting means furthermore comprise a supervisory apparatus (12), situated outside the body of the patient and having wireless communication means (14) for transmitting signals to the control unit (28).

2. Device according to Claim 1, **characterized in that** the pump (22, 44) is a volumetric pump.

3. Device according to Claim 1 or 2, **characterized by** an implantable control box (9) containing the electronic control unit (28) and the pump (22, 44).

4. Device according to Claim 3, **characterized in that** the control box contains the battery (37).

5. Device according to one of the preceding claims, **characterized by** means (47) for measuring the volume of the liquid transferred by the pump.

6. Device according to Claim 1, **characterized in that** the control unit (28) comprises means of wireless communication (31), the control unit (28) and the supervisory apparatus (12) being arranged so as to transmit information between themselves via their means of wireless communication (31, 14).

7. Device according to Claim 6, **characterized in that** the said information comprises a personal identification code (PIN) recorded in the control unit.

8. Device according to Claims 5 and 6, **characterized in that** the control unit (28) is arranged so as to transmit via the said means of wireless communication (31, 14) an indication of the volume of liquid transferred.

9. Device according to one of the preceding claims, **characterized by** an implantable pressure sensor (23), indicating to the control unit (28) the pressure of the liquid in the tube (7), the control unit (28) being arranged so as to produce an alarm signal via means of wireless communication (31) when the said pressure departs from a predetermined range.

10. Device according to Claim 9, **characterized in that** it furthermore comprises a monitor (13) situated outside the body of the patient and provided with means of wireless communication with the control unit (28), the monitor having display means (41) which are activated in response to the reception of the said alarm signal.

11. Device according to one of the preceding claims, **characterized in that** the pump (22) is able to operate selectively in opposite directions, so as to pump liquid either towards the gastric band (1), or towards the reservoir (11).

12. Device according to one of the preceding claims, **characterized in that** an electrovalve (21, 45) controlled by the control unit is wired in series between the reservoir (11) and the pump (22, 44).
